Europäisches Patentamt

European Patent Office

Office européen des brevets

(11) Veröffentlichungsnummer: **0 385 155**
**A2**

## (12) EUROPÄISCHE PATENTANMELDUNG

(21) Anmeldenummer: 90102500.7

(22) Anmeldetag: 08.02.90

(51) Int. Cl.⁵: **A61K 7/48**

(30) Priorität: 21.02.89 DE 3905299

(43) Veröffentlichungstag der Anmeldung:
05.09.90 Patentblatt 90/36

(84) Benannte Vertragsstaaten:
**AT BE CH DE DK ES FR GB GR IT LI LU NL SE**

(71) Anmelder: **Klein, Peter, Prof.Dr.med.**
**Oppeltshofer Weg 78**
**D-7980 Ravensburg(DE)**

(72) Erfinder: **Klein, Peter, Prof.Dr.med.**
**Oppeltshofer Weg 78**
**D-7980 Ravensburg(DE)**

(74) Vertreter: **Lederer, Franz, Dr. et al**
**Lederer, Keller & Riederer, Patentanwälte,**
**Lucile-Grahn-Strasse 22**
**D-8000 München 80(DE)**

(54) **Kosmetisches Präparat zur Behandlung der Haut.**

(57) Die Erfindung betrifft ein kosmetisches Präparat zur Behandlung der gesunden, intakten Haut enthaltend als Wirkstoffe Natriumchlorid, Kaliumchlorid, Magnesiumchlorid; $6H_2O$, Dinatriumhydrogenphosphat, Dextrose und L-Asparaginsäure.

## Kosmetisches Präparat zur Behandlung der Haut

Die Erfindung betrifft ein kosmetisches Präparat und die Verwendung einer speziellen pharmazeutischen Zusammensetzung zur Behandlung der Haut.

Die DE-A-23 51 512 beschreibt ein Arzneimittel in Form einer wäßrigen Lösung bestehend aus

| | |
|---|---|
| Natriumchlorid | 6,800 g |
| Kaliumchlorid | 0,170 g |
| Magnesiumchlorid; $6H_2O$ | 0,173 g |
| Dinatriumhydrogenphosphat | 1,280 g |
| Dextrose | 0,850 g |
| Asparaginsäure | 3,000 g |
| Sulfamethazine ( = Sulfadimidin) | 0,750 g |
| Phenolrot oder eine entsprechende Menge eines anderen Indikators | 0,075 g |
| Natriumhydroxid | 0,770 g |
| Wasser auf | 1000 ml |

Dieses Arzneimittel dient zur Behandlung von Verbrennungswunden, wobei eine Heilung der Wunde trotz Infektion ohne Zerstörung von noch lebenden Hautzellen erreicht wird. Durch die Applikation dieser Lösung soll eine Wundheilung dadurch erreicht werden, daß die Vermehrung der Hautzellen gefördert wird. Noch lebende Hautzellen auf der Wundfläche werden zum Wachstum angeregt, wobei von den noch lebenden Hautzellen als Inseln Hautwucherungen ausgehen.

Eine Wundlösung ähnlicher Zusammensetzung zur Feuchtbehandlung bei Epithelschäden der Haut, die kommerziell angeboten wurde, enthielt gemäß Firmenschrift

| | |
|---|---|
| Natriumchlorid | 6,800 g |
| Natriummonohydrogenphosphat | 3,230 g |
| Kaliumchlorid | 0,170 g |
| Magnesiumchlorid ($6H_2O$) | 0,173 g |
| Glucose-Monohydrat | 0,940 g |
| Mononatrium-L-aspartat ($1H_2O$) | 3,330 g |
| L-Asparaginsäure | 0,440 g |
| Sulfadimidin | 0,750 g |
| Phenolsulfonphthalein (Phenolrot) | 0,0075 g |
| Wasser auf | 1 Liter |

Diese Wundlösung wurde empfohlen zur Behandlung von Epitheldefekten der Haut, insbesondere nach Verbrennungen aller Schweregrade, Verbrühungen, Verätzungen, Abschürfungen, schlecht heilenden chronischen Wunden wie Ulcus cruris, Decubitalulcus, Strahlenulcus und Läsionen nach Psoriasiserkrankungen, sowie zur Vorbereitung auf Hauttransplantationen.

In der Anwendungsvorschrift heißt es ausdrücklich, daß die Applikation der Wundlösung auf die Wunde zu beschränken ist und daß zur Schonung des intakten Epithels des Wundrandes dieses mit einer externen Grundlage abgedeckt werden sollte. Bei der Behandlung chronischer Wunden heißt es ausdrücklich, daß der Hinweis auf die Schonung der intakten Haut des Wundrandes zu beachten ist. Bei Verbrennungen, die sich nur in einer Rötung der Haut manifestieren, ist eine Behandlung mit dieser Wundlösung nicht nötig.

Ein proliferationsstimulierender Effekt, wie er bei dieser bekannten Wundlösung herausgestellt wird, wird bei der Behandlung gesunder Haut nicht angestrebt.

Aufgabe der vorliegenden Erfindung war es eine pharmazeutische Zubereitung zur Verfügung zu stellen, die sich zur kosmetischen Behandlung, nämlich zur erhaltenden, vorbeugenden und verbessernden Pflege von gesunder, intakter Haut eignet, insbesondere solcher, die durch Körperausscheidungen, Witterungs-, Umwelt- oder Lichteinflüsse beansprucht ist.

Zur Behandlung von Entzündungen jeder Art, insbesondere bei durch übermäßige Sonneneinstrahlung oder Nahrungsmittel entzündlich angegriffener Haut werden vielfach Cortisonhaltige Präparate eingesetzt. Ziel der Erfindung war es, nicht unbedenkliche Wirkstoffe solcher Art zu vermeiden.

Die Verwendung einer Zusammensetzung gemäß der oben geschilderten Wundlösungen bot sich aus zweifachen Gründen nicht an. Einmal war ein übermäßiges Wachstum der Hautzellen wie es bei der Wundlösung gewünscht war nicht anzustreben, zum anderen enthielten diese bekannten Zusammensetzungen als wirksamen Kombinationsbestandteil Sulfadimidin, also ein Sulfonamid, und Sulfonamide neigen bekanntlich dazu unerwünschte Hautreaktionen auszulösen und sollten daher in kosmetischen Mitteln vermieden werden.

Es wurde nun überraschenderweise gefunden, daß eine Zusammensetzung, wie sie in den bekannten Wundlösungen vorliegt, jedoch kein Sulfonamid enthält, eine hervorragende hautaktivierende Wirkung aufweist durch die Auflockerung der bestehenden Epithelstrukturen und die Verbesserung der Durchblutung im Capillarbereich, insbesondere die schnellere Erneuerung verbrauchter Hautzellen, und sich in ausgezeichneter Weise zur kosmetischen Behandlung von gesunder, intakter Haut eignet.

Gegenstand der Erfindung ist somit ein kosmetisches Präparat zur Behandlung der gesunden, intakten Haut enthaltend als Wirkstoffe in wäßrigem Medium

| | |
|---|---|
| Natriumchlorid | 2 - 40 ‰, vorzugsweise 4 - 10 ‰ |
| Kaliumchlorid | 0,1 - 2 ‰, vorzugsweise 0,1 - 0,2 ‰ |
| Magnesiumchlorid; $6H_2O$ | 0,1 - 2 ‰, vorzugsweise 0,1 - 0,2 ‰ |
| Dinatriumhydrogenphosphat | 0,5 - 10 ‰, vorzugsweise 1,0 - 4,0 ‰ |
| Dextrose | 0,2 - 5 ‰, vorzugsweise 0,5 - 1 ‰ |
| L-Asparaginsäure | 1 - 20 ‰, vorzugsweise 2 - 10 ‰ |
| Natriumhydroxid | 0 - 5 ‰, vorzugsweise 0 - 1 ‰ |

Der pH-Wert des Präparates sollte im Bereich von 6,8 bis 7,0 liegen, vorzugsweise 6,9 betragen.

Die L-Asparaginsäure kann als solche eingesetzt werden und dann zur Erzielung des gewünschten pH-Wertes mit Natriumhydroxid in Salzform übergeführt werden, oder sie kann vollständig oder teilweise in Salzform, beispielsweise als Mononatrium-L-aspartat eingesetzt werden.

Die in Promille ausgedrückten Mengenanteile beziehen sich auf das Gewicht. Sie entsprechen einer Menge in Gramm auf ein Liter wäßrige Lösung.

Obwohl das Präparat als wäßrige Lösung eingesetzt werden kann, empfiehlt es sich zur besseren Applikation eine Salben- oder Cremeform zu wählen, wobei eine wäßrige Fettemulsion als Basis eingesetzt werden kann. Geeignete Fettemulsionen sind solche, wie sie üblicherweise in kosmetischen Hautcremes Verwendung finden.

Eine besonders bevorzugte Zusammensetzung enthält

| | |
|---|---|
| Natriumchlorid | 6,8 ‰ |
| Kaliumchlorid | 0,17 ‰ |
| Magnesiumchlorid; $6H_2O$ | 0,173 ‰ |
| Dinatriumhydrogenphosphat | 3,23 ‰ |
| Glucose-Monohydrat | 0,9 ‰ |
| Mononatrium-L-aspartat, $1H_2O$ | 3,3 ‰ |
| L-Asparaginsäure | 0,4 ‰ |

Es empfiehlt sich, den Präparaten außerdem einen pH-Indikator zuzusetzen, beispielsweise Phenolrot, welches in der bevorzugten Zusammensetzung in einer Menge von 0,075 % eingesetzt werden kann, wobei ein Farbumschlag anzeigt, daß das Präparat nicht mehr gebrauchsfähig ist.

Beispiel

Die Herstellung eines erfindungsgemäßen Präparates in Form einer wäßrigen Lösung wird nachstehend beschrieben.

Zur Herstellung eines Gesamtvolumens von 1000 ml werden zunächst 3,0 g Asparaginsäure in ca. 200 ml kochendem Wasser gelöst. In einem getrennten Gefäß werden in ca. 330 ml Wasser 0,77 g Natriumhydroxid gelöst. In dieser Lösung werden nacheinander 6,8 g Natriumchlorid, 0,17 g Kaliumchlorid, 0,173 g Magnesiumchlorid, 6H$_2$O, 1,28 g Dinatriumhydrogenphosphat und 0,85 Dextrose gelöst. Der so hergestellten Lösung wird die noch heiße Lösung von Asparaginsäure in Wasser zugefügt, zuletzt wird gegebenenfalls der Indikator, z.B. 0,075 g Phenolrot beigegeben. Die nun vorhandene Lösung läßt man auf Raumtemperatur abkühlen und füllt mit destilliertem Wasser auf 1000 ml auf.

Gewünschtenfalls kann die Lösung noch sterilisiert werden, z.B. durch Erhitzen im Autoklaven auf 120 °C während 20 Minuten.

**Ansprüche**

1. Kosmetisches Präparat zur Behandlung der gesunden, intakten Haut enthaltend als Wirkstoffe in wäßrigem Medium

| | |
|---|---|
| Natriumchlorid | 2 - 40 ‰, vorzugsweise 4 - 10 ‰ |
| Kaliumchlorid | 0,1 - 2 ‰, vorzugsweise 0,1 - 0,2 ‰ |
| Magnesiumchlorid; 6H$_2$O | 0,1 - 2 ‰, vorzugsweise 0,1 - 0,2 ‰ |
| Dinatriumhydrogenphosphat | 0,5 - 10 ‰, vorzugsweise 1,0 - 4,0 ‰ |
| Dextrose | 0,2 - 5 ‰, vorzugsweise 0,5 - 1 ‰ |
| L-Asparaginsäure | 1 - 20 ‰, vorzugsweise 2 - 10 ‰ |
| Natriumhydroxid | 0 - 5 ‰, vorzugsweise 0 - 1 ‰ |

2. Kosmetisches Präparat nach Anspruch 1 enthaltend als Wirkstoffe

| | |
|---|---|
| Natriumchlorid | 6,8 ‰ |
| Kaliumchlorid | 0,17 ‰ |
| Magnesiumchlorid; 6H$_2$O | 0,173 ‰ |
| Dinatriumhydrogenphosphat | 3,23 ‰ |
| Glucose-Monohydrat | 0,9 ‰ |
| Mononatrium-L-aspartat, 1H$_2$O | 3,3 ‰ |
| L-Asparaginsäure | 0,4 ‰ |

3. Kosmetisches Präparat nach Anspruch 1 oder 2 auf Basis einer wäßrigen Fettemulsion.
4. Kosmetisches Präparat nach Anspruch 3 in Salben- oder Cremeform.
5. Verwendung einer Wirkstoffkombination nach Anspruch 1 oder 2 zur Herstellung von kosmetischen Präparaten zur Behandlung der gesunden, intakten Haut.